# EUROPEAN PATENT APPLICATION

(11) **EP 3 819 350 A1**
(43) Date of publication of application: **12.05.2021**
(21) Application number: 20386049.9
(22) Date of filing: 01.10.2020
(51) Int. Cl.: C09J 7/29, C09J 107/02, A61F 13/02

(54) **MULTILAYER-MULTIFUNCTIONAL TAPE**

(30) Priority: 05.11.2019 GR 20190100494
(71) Applicant: Koufos, Aimilios, 15344 Gerakas (Attica) (GR)
(72) Inventor: Koufos, Aimilios, 15344 Gerakas (Attica) (GR)

(57) **Abstract**

Multilayer - multifunctional tape in form of bandage, adhesive, hypoallergenic, in various colors and shapes, 0,2 - 0,4 mm thick, 1,5 - 5 cm wide, in rolls 1 - 25 m long. It has a limited stiffness of 90% in width and length and 95% in width (semi-hard) and length in a second version of the tape (hard), having two degrees of freedom and can be adjusted on geometrical changes and curves of the human body. Made of two layers of cotton with transversal fibers and adhesive hypoallergenic material. One layer of cotton is impregnated with natural hypoallergenic, antimicrobial, antirheumatic, antihistaminic substances. In addition, it has on the weld area anti-adhesive coating which is also waterproof and can be easily detached. The tape has a supportive effect on musculoskeletal conditions and chronic diseases, contributing in recovery, while its bioactive substances accelerate healing and relieve pain, increasing the reflex action of the skin towards the cortex of the brain.

## Description

The said invention consists of a multilayer - multifunctional two-dimensional limited elasticity adhesive tape - bandage that is made of 100% hypoallergenic material and adhesive coating that is also hypoallergenic. The tape is impregnated with bioactive substances that act upon rehabilitation of musculoskeletal and chronic diseases. It also has a pain-relieving effect on joint discomforts caused by rheumatic diseases. The multilayer tape achieves its purpose based on its special two-dimensional limited elasticity and its special biological and bioactive substances that contains. It is as thin as skin. Its main material is the 100% hypoallergenic natural cotton with cross fibers and the adhesive tape that is also hypoallergenic and does not contain substances that cause dermatitis. It has a thickness of 0,2 to 0,3 mm and it shall be available in bandage-type rolls of length from 1 to 25 m and width of 1,5 cm, 3,5 cm up to 5 cm. It shall be manufactured in two forms of elasticity: (1). >90% rigid on the longitudinal axis and >90% on the transverse (2). >95% rigid on the longitudinal axis and >95% on the transverse. The tape is covered along its length by an anti-adhesive paper that protects the fabric, the adhesive and the impregnation layer of bioactive therapeutic substances. Therapeutic substances that it may contain in whole or in part are: 1. Capsaicin, 2. Glycol Salicylate, 3. Eucalyptus oil, 4. Camphora, 5. Methol, 6. Eugenol, 7. Thymol. These substances have antimicrobial, anti-inflammatory, anti-itch, antihistaminic and analgesic effect on the area where the multilayer Polyfix Taping will be applied. When easily applied upon the skin surface it can be curved due to its two-dimensional limited elasticity and thus take the shape of soft tissues of the human body.

It is easily cut with scissors. It is permeable by air, water and to sunlight, so that no problem would be caused to skin, such as drying and discoloration of the skin. To be applied for 2-3 days without change but in case of need it may remain on the skin surface up to 7 days, preserving its effectiveness.

The essense of the invention is the following:
Existing tapes will be very flexible or inelastic and may not be placed directly on the skin on their own, since the tapes contain latex (polyisoprene or natural rubber), which causes dermatitis when in contact with the skin surface. For this reason a hypoallergenic gauze (Fixomull) is placed first on the skin and then the Leukotape that contains latex. This is the most important disadvantage because the mechanical stability of the material is affected by the rigid behaviour of the Leukotape. As a result of this, the existing material and technique of taping restrict the movement of the underlying tissues.

First disadvantage of the said material is that it may not be applied in the same way unto curvy parts or parts with intense geometric changes of the human body, such as foot or knee joint or quadricepts muscles etc.

The most important advantage of the invention is that is the replacement of the said material (Leukotape and Fixomull) and the other elastic tapes by a single material that has two degrees of freedom: the longitudinal and transverse of the axis and can be adjusted on parts with geometric changes and curves of the human body. In this way its supportive effect on soft tissue injuries is achieved, including also the articulated surfaces.

The second advantage of this invention is its therapeutic effect through the transdermal release of some or all of the following substances that is achieved in the long term:
1. Capsaicin, 2. Glycol Salicylate, 3. Eucalyptus oil, 4. Camphora, 5. Methol, 6. Eugenol, 7. Thymol. The above known, safe and internationally approved substances (NOM, FDA, EMA) have antimicrobial, anti-inflammatory, anti-itch, antihistaminic, antirheumatic and analgesic effect on the area where the multilayer - multifunctional Polyfix Taping will be applied. These substances are located in a special pre-impregnated layer between the adhesive layer and the hypoallergenic cotton layer. The tape shall have a width of 1,5 cm, 3,5 cm to 5 cm and length of 1 to 25 m in a roll. It shall be manufactured in two forms of elasticity: (1). >90% rigid on the longitudinal axis and >90% on the transverse (semi-hard). (2). >95% rigid on the longitudinal axis and >95% on the transverse (hard).

Its especially high rigidity is required to provide support to the underlying soft tissues of the body and to encourage their recovery. It alows limited extent in the transversal direction in order to be able to adjust on geometric changes and curves of the human body.

Its elasticity shall be absolutely far from any other type of elastic materials and tapes that we know which are 100% rigid in both directions. Also many of these materials contain latex which causes allergic dermatitis.

### Advantages of the invention

The advantages of the patent are the following compared with the relevant state of the art.
1. Therapeutic and supporting effect for musculoskeletal injuries is being achieved, by means of using one single material;
2. It is hypoallergenic compared to other materials;
3. Skin breaths much better thanks to its structure and active bioactive substances of the tape;
4. The tape and glue are waterproof; it also can be used at sea by the althlete;
5. Time of application and use by the specialist therapist is faster;
6. Two-dimensional limited elasticity for application in difficult areas of the body, such as knee, foot joint, scapula, etc, resulting in their stabilization;
7. It may remain on skin surface up to 7 days in case of need, without loosing its effect, saving considerable time and materials;
8. It can be used in rheumatic and chronic pains except for soft tissue injuries, having a pain-relieving effect;
9. The cost of the purchase is less;
10. It is made of ecological and organic products and bioactive substances;
11. It provides the althlete with the possibility to apply it on his own in some parts of the body, in case of need, by using simple instructions on how to use the material.

### Technical field of the invention

The technical field of the patent refers to materials that tapes areas of the human body, appropriate for the treatment and rehabilitation of soft tissue injuries. This invention is composed of three layers, one hypoallergenic cotton, one impregnated with bioactive substances and one adhesive hypoallergenic tape with waterproof paper for protection. It has a two-dimensional limited flexibility and can be adjusted on geometric changes and curves of the human body. The limited flexibility provides a supportive effect.

### State of the Art

The multilayer - multifunctional Tape «Polyfix Taping» is applied externally to the human body, never to open wounds but only to skin without incisions. It helps to stabilize the joints after injury or surgery and contribute in their recovery by achieving better mechanical motion and offering a psychological boost to the patients, especially those suffering from sport injuries. It has antihistaminic, anti-inflammatory, antirheumatic and antimicrobial effect thanks to its biological and bioactive substances.

The said material is characterized by the possibility of being easily adjusted while applied even unto curves and anatomical changes of the human body, such as in joints. When properly applied it has a mechanical effect on the joints, providing mechanical support and a safety sense. In the case of further stabilization of a joint, the tape promotes the function of musculoskeletal system ensuring the required limitations of movement and the correlation between force and extension. The placement of the tape on a muscle externally in combination with its bioactive substances stimulate the mechanoreceptors of the skin that, in turn, will control the activity of the musculoskeletal system. Moreover, its bioactive substances are gradually released and speed recovery of soft tissue injuries by stimulating the Peripheral Nervous System and the Central Nervous System.

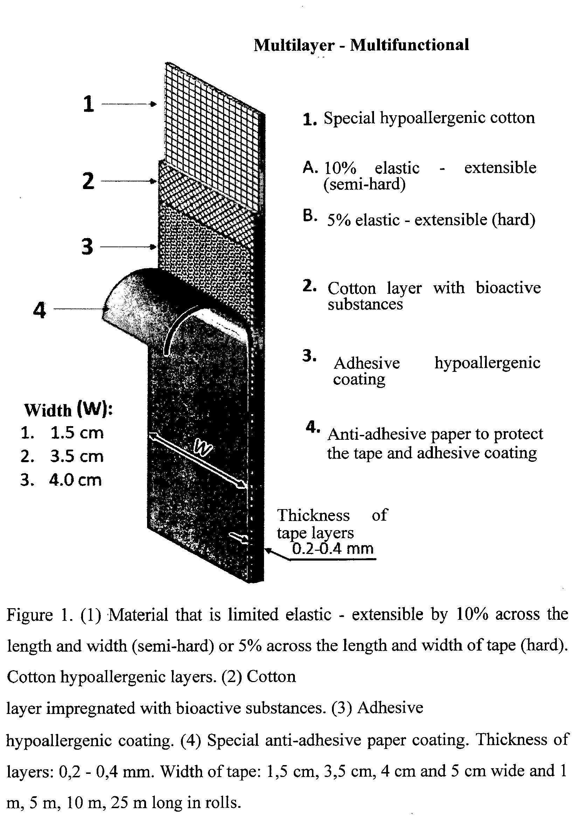

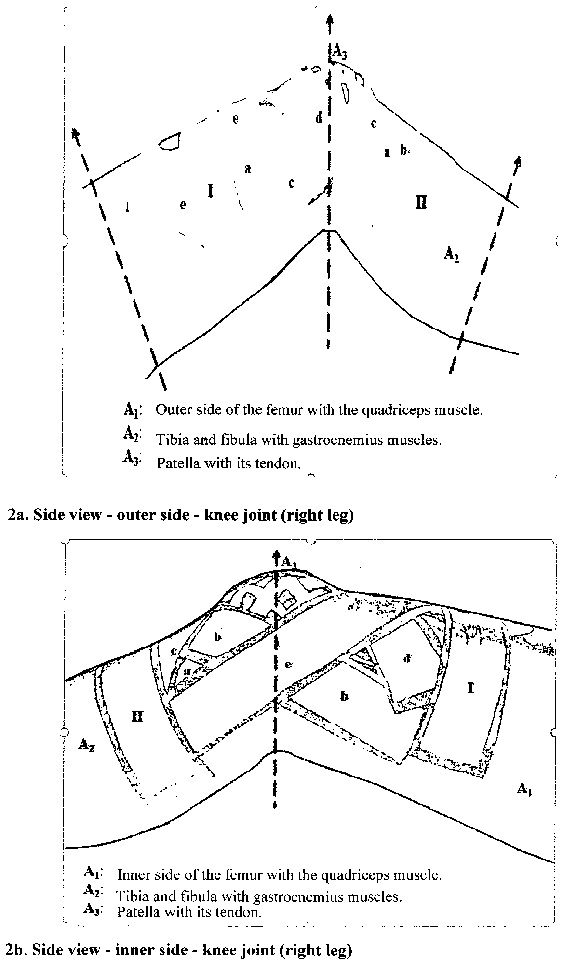

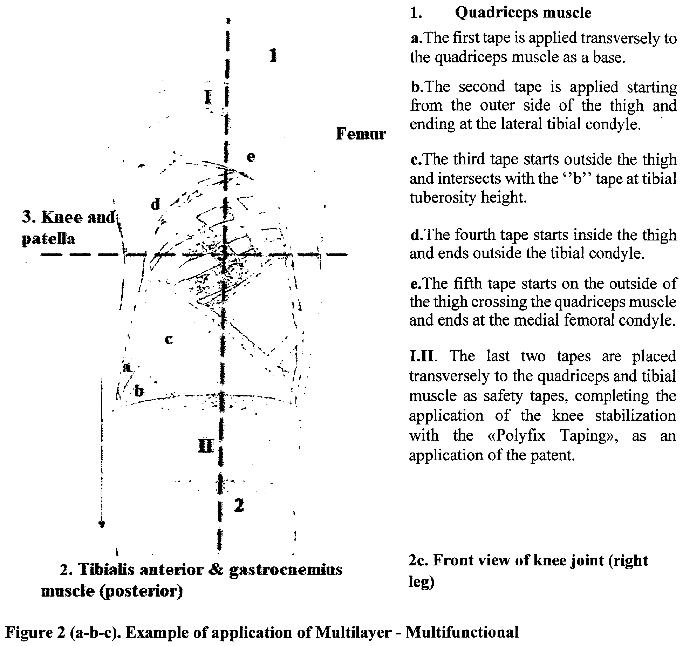

## Claims

1. Three layer tape: a layer of latex free hypoallergenic adhesive, a layer of dense cotton impregnated with bioactive substances and one of cotton with transversal fibers waterproof.

2. The said material has two different forms of rigidity >90% on the longitudinal axis and on the transverse (semi-hard) and >95% on the longitudinal axis and on the transverse (hard).

3. Based on Claim 1, thanks to hypoallergenic properties the tape can remain on the skin for five to seven days (5-7).

4. Based on Claim 1, the tape may contain all or some of the following bioactive natural substances: 1. Capsaicin, 2. Glycol Salicylate, 3. Eucalyptus oil, 4. Camphora, 5. Methol, 6. Eugenol, 7. Thymol.

5. Based on Claim 2, the tape can be easily adjusted on anatomical peculiarities and geometric changes of articular cavities and muscles of the human body.
